## Europäisches Patentamt
## European Patent Office
## Office européen des brevets

(11) Veröffentlichungsnummer: **0 431 548 B1**

## EUROPÄISCHE PATENTSCHRIFT

(45) Veröffentlichungstag der Patentschrift: **22.02.95**

(51) Int. Cl.6: **C12N 9/88**, //C12P5/00, (C12N9/88,C12R1:44)

(21) Anmeldenummer: **90123194.4**

(22) Anmeldetag: **04.12.90**

(54) Fructose-1,6-bisphosphat-Aldolase, Verfahren zur Herstellung derselben und deren Verwendung.

(30) Priorität: **07.12.89 DE 3940431**
**21.08.90 DE 4026382**

(43) Veröffentlichungstag der Anmeldung:
**12.06.91 Patentblatt 91/24**

(45) Bekanntmachung des Hinweises auf die Patenterteilung:
**22.02.95 Patentblatt 95/08**

(84) Benannte Vertragsstaaten:
**CH DE DK FR GB LI NL**

(56) Entgegenhaltungen:

**European Journal of Biochemistry vol. 108, 1980, Berlin, pages 295-301; GÖTZ F. et al: "Purification and characterization of an unusually Heat-stable and acid/base-stable class 1 Fructose-1,6-bisphosphate aldolase from Staphylococcus aureus"**

(73) Patentinhaber: **FORSCHUNGSZENTRUM JÜLICH GMBH**
**Wilhelm-Johnen-Strasse**
**D-52425 Jülich (DE)**

(72) Erfinder: **Brockamp, Hans-Peter**
**Keltenstrasse 23**
**W-5162 Niederzier (DE)**
Erfinder: **Kula, Maria-Regina**
**Selgenbusch 12**
**W-5162 Niederzier (DE)**
Erfinder: **Götz, Friedrich**
**Beim Herbstenhof 31**
**W-7400 Tübingen (DE)**

Rank Xerox (UK) Business Services
(3. 10/3.09/3.3.3)

International Journal of Systematic Bacteriology, vol. 33, no. 3, July 1983, Washington,D.C., pages 443 - 450; FISCHER S. et al.: "Immunochemical and Protein-chemical Studies of class 1 Fructose-1,6-Diphosphate Aldolases from Staphilococci"

Journal of Biological Chemistry, vol. 248, no. 5, 10 March 1973, Baltimore, US, pages 1650 - 1659; LEBHERZ H.G. et al: "A class I (Schiff Base) Fructose Diphosphate Aldolase of Prokariotic Origin"

M. Schultz et al., Tetrahedron Letters, vol. 31, pp. 867-868, 1990

F. Götz et al., Microbiol. Letters, vol. 5, pp. 253-257, 1979

K.H. Schleicher et al., Intern. Journal of Systemat. Bacteriology, vol.32, pp. 153-156, 1982

**Beschreibung**

Die Erfindung bezieht sich auf ein Verfahren zur Synthese von Kohlenhydraten bzw. Derivaten derselben durch enzymatische Umsetzung von Aldehyden mit Dihydroxyacetonphosphat in Gegenwart von aus Staphylococcen isolierter Klasse 1-Fructose-1,6-bisphosphat-Aldolase (F-1,6-BP-Aldolase) und sie umfaßt ein Verfahren zur Gewinnung einer solchen Aldolase und das damit erhältliche Enzym.

Das aktuelle Interesse an gezielten Aldolreaktionen und an der Synthese chemisch schwer zugänglicher bzw. in der Natur nicht vorkommender Monosaccharide führte dazu, daß den Aldolasen in diesem Zusammenhang mehr und mehr Beachtung geschenkt wurde. Für die organische Synthese dieser biologisch wichtigen Substanzen, die auch für die Medizin von Nutzen sein können, bietet die Verwendung von Biokatalysatoren eine effektive Alternative. Die F-1,6-BP-Aldolase des Kaninchenmuskels ist bislang das einzige Enzym, das in der Synthese angewandt wird (A. E. Seriani u. a. Meth. Enzymol. 89 (1982) 83-92). Diese aus Kaninchenmuskel isolierte Aldolase ist teuer und relativ instabil.

Andererseits sind Aldolasen als Enzyme des Glycolyse-Stoffwechsels ubiquitär und ihre Existenz in Micrococcen und Staphylococcen seit langem bekannt.

So wird gemäß H.G.Lebherz et al. (J.Biol.Chem. 248 (1973) Seiten 1650-59) Klasse 1 F-1,6-BP-Aldolase von Micrococcus aerogenes gebildet. F.Götz et al.(FEMS Microbiol.Letters 5 (1979) 253-57)berichten über die Verteilung von Klasse 1 und Klasse 2 F-1,6-BP-Aldolasen in unterschiedlichen Staphylococcen, Peptococcen und Micrococcen. In 1980 wurde von F.Götz et al. (EUr.J.Biochem. 108 (1980)293-301) über eine aus Staphylococcus aureus isolierte Klasse 1 F-1,6-BP-Aldolase mit ähnlichen Eigenschaften wie die aus Kaninchenmuskel isolierte Aldolase jedoch mit verbesserter Thermostabilität detailliert berichtet, die allerdings wegen der pathogenen Eigenschaften von Staphylococcus aureus als Enzymproduzent offensichtlich keine praktische Bedeutung erlangt hat.

Auch der Bericht von S.Fischer et al. (Internat.J.System. Bacteriol. 33 (1983) 443-50) über die Isolierung von Klasse 1 F-1,6-BP-Aldolasen aus zahlreichen unterschiedlichen Staphylococcen hat offensichtlich keine Beachtung im Bereich der Kohlenhydratsynthese gefunden, wie aus der Arbeit von M.Schultz et al. (Tetrahedran Letters 31 (1990)867-68) hervorgeht, in der noch 1990 über stereospezifische Reaktionen mit "Kaninchenmuskel-Aldolase" berichtet wird.

Ziel der Erfindung ist daher eine Kohlenhydratsynthese mit einer in ihren Eigenschaften detailliert beschriebenen Aldolase mit verbesserter Zugänglichkeit gegenüber den bislang für solche Synthesen verfügbaren F-1,6-BP-Aldolasen aus Kaninchenmuskel oder Staphylococcus aureus und einer für enzymkatalysierte Prozesse befriedigenden Stabilität.

Dieses Ziel wird erfindungsgemäß dadurch erreicht, daß man eine aus Staphylococcus carnosus isolierte F-1,6-BP-Aldolase verwendet.

Diese Aldolase zeichnet sich durch eine im Vergleich zur "Kaninchenmuskel-Aldolase" erheblich verbesserte Stabilität aus entsprechend einer Desaktivierungsrate unter Synthesebedingungen bei T = 25 °C; pH 6,5; 20 mM DHAP; 75 mM Glyoxylsäure; Enzymaktivität 2,9 U/ml bzw. 25 U/mg von ≤ 1 %/d (z.B. gefunden: 0,77 %/d) bzw. einer 60 °C- bzw. 100 °C-Stabilität in aufgereinigter Form (≈25 U/mg) bei pH 7,5 in 0,06 M Tris-HCl-Pufferlösung in Eppendorfgefäßen entsprechend einer Restaktivität nach 30 Minuten zwischen 70 und 80 % bzw. 20 und 30 %, insbesondere von 76 % bzw. 26 %, und sie hat damit eine nur etwas geringere thermische Stabilität als die Aldolase aus Staphylococcus aureus (letztere zeigt nach 90 min bei 100 °C keinen merklichen Aktivitätsverlust), ohne mit deren gravierendem Nachteil opportunistisch pathogener Herkunft behaftet zu sein.

Der als Enzymproduzent benutzte Staphylococcus carnosus ist aus natürlichen Quellen, insbesondere aus trockener Rohwurst ohne weiteres zugänglich und in Form von Starterkulturen im Handel erhältlich (siehe K.H.Schleifer et al., Internat.J.System.Bacteriol. 32 (1982) 153-56). Von Schleifer et al. wird zwar u.a. die Bildung von Klasse 1 F-1,6-BP-Aldolase durch S. carnosus erwähnt, die jedoch weder isoliert noch in ihren spezifischen Eigenschaften näher untersucht oder beschrieben wurde.

Im Rahmen der vorliegenden Erfindung wurde insbesondere der unter der Nummer DSM 20 501 bei der Deutschen Sammlung für Mikroorganismen in Göttingen hinterlegte Staphylococcus carnosus-Stamm verwendet.

Die Anzucht des Stammes erfolgt in Schüttelkulturen in 500 ml Erlenmeyerkolben mit 200 ml Medium bei 110 rpm bzw. im Fermenter mit einem Arbeitsvolumen zwischen 5 und 10 l. Das Kulturmedium (M 1) hatte z.B. folgende Zusammensetzung:

1,0 % Trypton
0,5 % Hefeextrakt
0,5 % NaCl
0,1 % $Na_2HPO_4$

3

EP 0 431 548 B1

1,0 % Glucose
pH 7,2

Zur Korrektur des pH-Wertes wurden 3M NaOH und 3M $H_3PO_4$ verwendet. Sofern nicht anders angegeben, wurden während der Fermentation folgende Parameter eingehalten:

| | |
|---|---|
| Drehzahl | 100 rpm |
| Temperatur | 37°C |
| Belüftung | 5 % $pO_2$ |
| Nährlösung | M1 |
| pH | 7,2 |
| Kulturdauer | 8 – 13 h |

Die Zellen wurden am Ende der logarithmischen Phase durch 10 minütige Zentrifugation bei 8000 rpm geerntet. Der Zellaufschluß erfolgte durch Naßvermahlung mit Glasperlen.

Das Feuchtgewicht der Biomasse aus 9 l Fermentationsvolumen betrug 121 g. Die nachfolgend angegebenen Reinigungsschritte wurden zwischen 1 und 6°C in 0,06 M Tris-HCl-Puffer bei pH 7,5 durchgeführt.

Zur Aufreinigung des Enzyms wurde eine fraktionierte Ammoniumsulfatfällung, eine pH-Fraktionierung und Ionenaustauschchromatographie vorgenommen:

Fraktionierte Ammoniumsulfatfällung

195 ml zellfreier Rohextrakt wurden mit festem Ammoniumsulfat bis zu einer Sättigung der Lösung von 40 % versetzt. Der pH-Wert wurde mit 3 M Ammoniak auf 7,5 eingestellt und die Fällung für zwei Stunden im Eisbad durchgeführt. Die ausgefallenen Bestandteile wurden durch 20 minütiges Zentrifugieren bei 20000 rpm abgetrennt. Schrittweise wurde dann die $(NH_4)_2SO_4$-Konzentration auf 60 % bzw. 80 % Sättigung erhöht, wiederum im Eisbad gefällt und zentrifugiert. Zur Ausfällung der Aldolase wurde der Überstand auf 100 % $(NH_4)_2SO_4$-Sättigung gebracht und der pH mit 7 M Essigsäure auf 5,0 gesenkt. Der Niederschlag wurde in 50 ml Aufschlußpuffer (0,06 M Tris-HCl pH 7,5 mit 6 mM 2-Mercaptoethanol) resuspendiert.

pH-Fraktionierung

Der pH-Wert von 50 ml der 100 % $(NH_4)_2SO_4$-Fraktion wurde unter starkem Rühren tropfenweise mit 7 M Essigsäure zunächst auf 4,0, dann mit 6 M HCl auf 3,5 und schließlich auf 3,0 gesenkt. Der gebildete Niederschlag wurde jeweils bei 20000 rpm 20 min lang abzentrifugiert. Die Aldolase befand sich im Überstand, welcher mittels Diafiltration mit einer Ultrafiltrationsmembran Amicon YM 10 entsalzt wurde.

Ionenaustauschchromatographie

30 ml der entsalzten Enzymlösung wurden auf eine 160 ml DEAE-Sephadex A 25 Säule aufgebracht, die zuvor mit 0,1 M NaCl enthaltendem 0,06 M Tris-HCl-Puffer von pH 7,5 gespült worden war. Das Enzym wurde durch einen linearen Salzgradienten von 0,1 - 0,5 M NaCl in 0,06 M Tris-HCl Puffer pH 7,5 eluiert. Die Flußrate betrug 30 ml/h. Die aktiven Fraktionen wurden vereinigt und gefriergetrocknet.

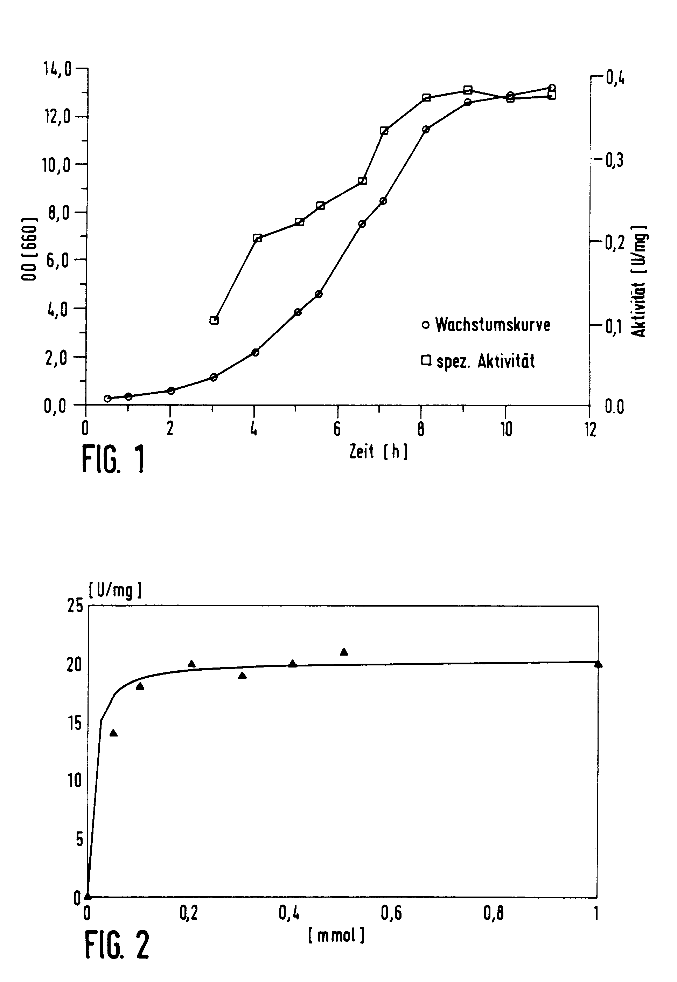

Das beigefügte Diagramm (Figur 1) zeigt Kurven für Wachstum und Enzymproduktion in Abhängigkeit von der Kulturdauer im Fermenter (5 % $pO_2$).

4

Die nachfolgende Tabelle 1 zeigt eine Übersicht über die Ergebnisse der Aufreinigung.

Tabelle 1

| | spezifische Aktivität [U/mg] * | Reinigungs- faktor | Ausbeute [%] |
|---|---|---|---|
| Rohextrakt | 0,35 | 1 | 100 |
| $(NH_4)_2SO_4$ | 1,6 | 4,6 | 43 |
| pH-Frakt. | 5,3 | 15,2 | 31 |
| DEAE-Chrom. | 25,0 | 71,4 | 21 |

* Protein bestimmt nach Bradford (Lit)

Alternativ kann die Aldolase, besonders zweckmäßig nach Zellernte und -aufschluß, durch wäßrige 2-Phasenextraktion von Biomasseballast getrennt werden.

Dazu dienen insbesondere Polymer/Salzgemische und speziell Polyethylenglycol/Kaliumphosphatmischungen. Andere Salze, wie z.B. Sulfat, sind gleichermaßen anwendbar. Besonders zweckmäßig ist ein hoher Gehalt an PEG mit kleinem Molekulargewicht, insbesondere unter 1500 Dalton.

Das Enzym sammelt sich in der Oberphase und kann in einem weiteren Schritt daraus rückextrahiert werden; technisch einfacher ist jedoch die unmittelbare Verwendung der Oberphase für eine nachfolgende Trennung über eine Anionenaustauschersäule. Eine solche unmittelbare Weiterverarbeitung ist ohne merkliche Nachteile möglich.

Als Säulenmaterialien eignen sich dafür Anionenaustauscher mit Aminoethyl-, Diethylaminoethyl- (DEAE-) und quaternären Aminoethylgruppen, die üblicherweise auf pH-Werte $\geq$ 5 und insbesondere auf pH 7,5 equilibriert worden sind. Insbesondere wurden DEAE Sephadex®, Mono Q und Q-Sepharose® verwendet.

Eluiert wird insbesondere mit eingestelltem Salzgehalt für fraktionierten Proteindurchlauf, z.B. mit einer 0,25 M Salzlösung mit Durchlaufgeschwindigkeiten von 3-5 ml/min.

Die gesammelten positiven Fraktionen, die das Enzym enthalten, können als solche oder in gefriergetrockneter Form verwendet werden.

Es folgt ein Beispiel für die Durchführung einer solchen Aufarbeitung mit wäßriger 2-Phasenextraktion: Aufarbeitung für S.carnosus Fructose-1,6-bisphosphat-Aldolase

I) Phasensystem
50 % Rohextrakt (40 % w/w)
27 % PEG 400
7 % $KP_i$ pH 7,5
Oberphase enthält 95 - 99 % der Enzymaktivität mit 0,8 U/mg Protein.

II) Q-Sepharose-Anionenaustauscher
Verwendet wurde eine 50 ml Säule equilibriert mit 20mM Tris-HCl Puffer pH 7,5
    0,15 M NaCl
    0,1 % Mercaptoethanol
Oberphase I wurde 4:1 verdünnt und auf den Austauscher gegeben.
Eluiert wurde mit 20 mM Tris-HCl pH 7,5
    0,25 M NaCl
    0,1 % Mercaptoethanol
    80 - 90 % Ausbeute; 7,1 U/mg Protein

III) Q-Sepharose-Anionenaustauscher
Equilibriert wurde wie unter II)
Positive Fraktionen aus Lauf I wurden 2:1 verdünnt auf den Austauscher gegeben.
Eluiert wurde das Protein mittels NaCl-Gradient von 0,15-0,25 M NaCl. Die Aldolase eluiert bei 0,2 M NaCl Konzentration.
70 - 80 % Gesamtausbeute; 13,1 U/mg Protein

Enzymcharakterisierung

Zur Charakterisierung des Enzyms wurden jeweils 40 $\mu$g/ml der aufgereinigten Aldolase eingesetzt, zur Molekulargewichtsbestimmung wurden 120 $\mu$g/ml verwendet.

Einstufung als Klasse I Aldolase

Aufgrund folgender Charakteristika kann die F-1,6-BP-Aldolase aus S. carnosus dem Klasse I Typ der Aldolasen zugeordnet werden.

- Die Aktivität gegenüber F-1,6-BP bleibt unbeeinträchtigt bei Anwesenheit verschiedener Konzentrationen EDTA.
- S. carnosus Aldolase wird bei Anwesenheit von DHAP und $NaBH_4$ vollständig inhibiert.
- Ein- und zweiwertige Kationen haben keinen Einfluß auf die Aldolase-Aktivität.

Weiterhin verhält sich die S. carnosus Aldolase sehr spezifisch gegenüber dem natürlichen Substrat.

Das Molekulargewicht der Aldolase wurde mittels SDS-PAGE bestimmt, und es wurden ~33000 Dalton (eine Untereinheit) ermittelt.

$K_m$-Werte nach Michaelis-Menten

Die $K_m$-Werte für die Substrate F-1,6-BP und F-1-P wurden über die Enzymaktivitäten bei verschiedenen Substratkonzentrationen ermittelt. Figur 2 zeigt den Michaelis-Menten-Plot für die Bestimmung des $K_m$-Werts von F-1,6-BP.

Dieser wurde zu 0,022 mM bestimmt, derjenige für F-1-P betrug 18,8 mM. Daraus wird deutlich, daß sich die Aldolase sehr spezifisch gegenüber dem natürlichen Substrat verhält. Der $K_m$-Wert für F-1-P ist um den Faktor 1000 größer als der für F-1,6-BP.

pH-Optimum

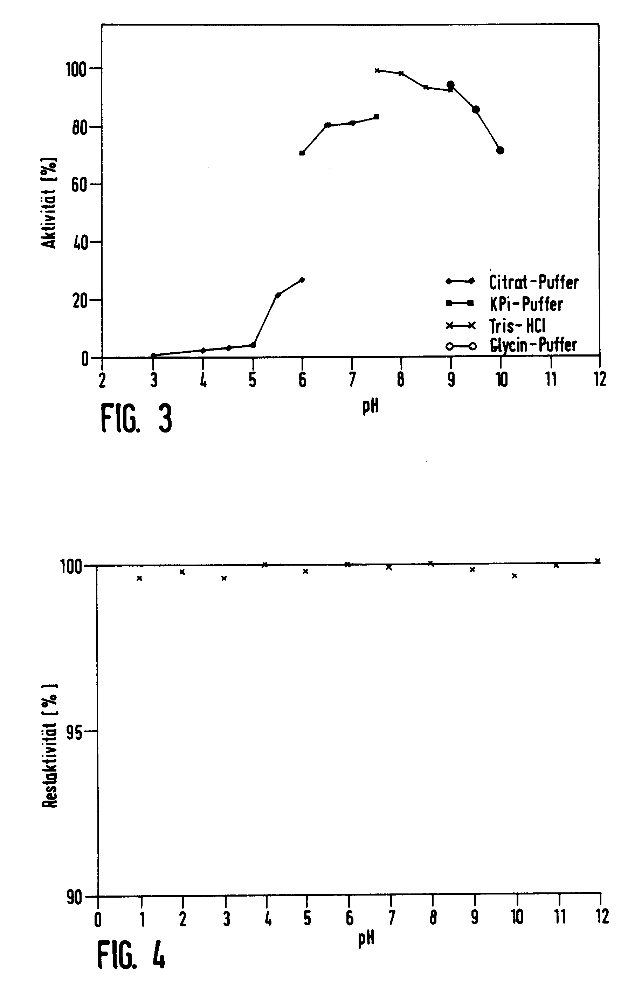

Zur Ermittlung des pH-Optimums (Fig. 3) wurde die Enzymaktivität der Aldolase bei 37°C zwischen pH 3 und pH 10 in folgenden Puffern gemessen:

```
0,06 M Citrat Puffer      pH 3,0 -  6,0
0,06 M KP -Puffer         pH 6,0 -  7,5
        i
0,06 M Tris-HCl Puffer    pH 7,5 -  9,0
0,06 M Glycin Puffer      pH 9,0 - 10,0
```

Aus Fig. 3 ist zweierlei erkennbar:

- Das pH-Optimum der Aldolase umfaßt den pH-Bereich 6,5 - 9,0 mit der Spitze bei pH 7,5.
- Das Enzym zeigt unterschiedliche Aktivitäten in Gegenwart der verschiedenen Puffer. Natriumcitrat-Puffer zeigt die stärkste Hemmung des Enzyms.

Stabilität der Aldolase

pH-Stabilität

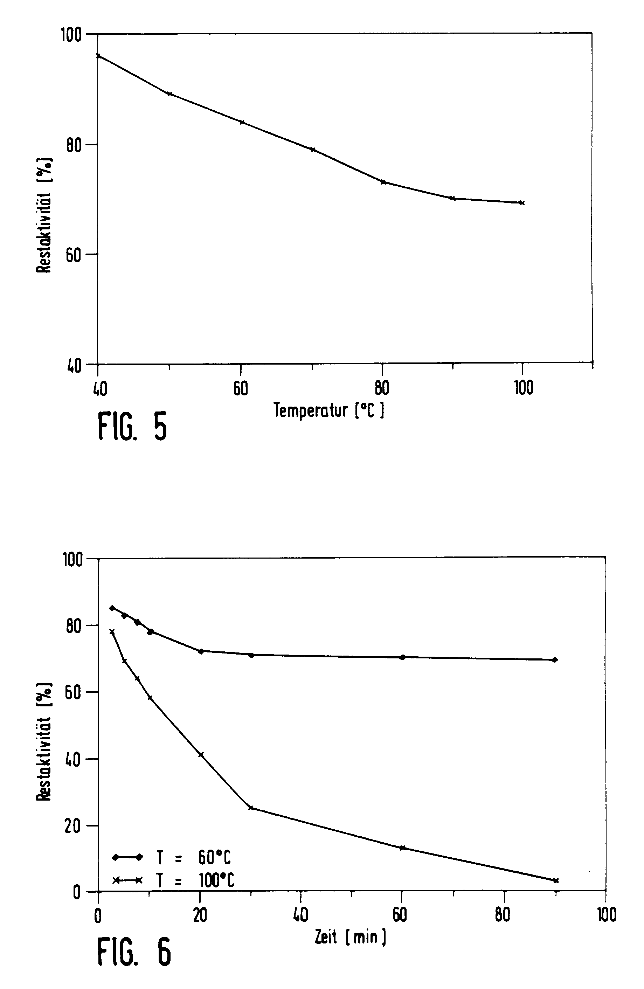

Die pH-Stabilität des Enzyms wurde durch zehnminütige Inkubation der Aldolase bei Zimmertemperatur (RT) zwischen pH 1 und pH 12 ermittelt (Fig. 4). Es wurde nur in 0,06 M Tris-HCl Puffer gearbeitet, wobei der jeweilige pH-Wert mit 2 N NaOH bzw. 2 N HCl eingestellt wurde. Nach Ablauf der Inkubationszeit wurden die Probenlösungen wieder auf pH 7,5 gebracht und die Enzymaktivitäten gemessen.

Wie Fig. 4 zeigt, hat die S. carnosus Aldolase eine sehr gute pH-Stabilität. In einem weiteren Test konnte festgestellt werden, daß die Aldolase-Aktivität auch nach 72 h Lagerung bei pH 2 und 4°C noch 100 % der Ausgangsaktivität betrug.

Temperatur-Stabilität

Zur Untersuchung der Temperatur-Stabilität der Aldolase wurden folgende Messungen durchgeführt:
1) Das Enzym wurde 5 min in 0,06 M Tris HCl Puffer von pH 7,5 bei Temperaturen zwischen 40°C und 100°C inkubiert und somit die Abhängigkeit der Enzymaktivität von der Temperatur bestimmt (Fig. 5).
2) In einer weiteren Testreihe wurden Proben der aufgereinigten Aldolase bei 60°C und 100°C unterschiedlich lange inkubiert und damit die Abhängigkeit der Enzymaktivität bei zwei verschiedenen Temperaturen von der Zeit bestimmt (Fig. 6).

Sowohl Fig. 5 als auch Fig. 6 zeigen, daß es sich bei der F-1,6-BP Aldolase aus S. carnosus um ein relativ Temperaturstabiles Enzym handelt. Sie weist zwar nicht die Hitzestabilität auf, die für die Aldolase aus S. aureus beschrieben ist (nach 90 min bei 100°C kein Aktivitätsverlust), übertrifft jedoch die Temperatur-Stabilität der Kaninchenmuskelaldolase bei weitem.

Lagerstabilität der Aldolase

Um Aussagen über die Lagerstabilität machen zu können, wurden Proben der aufgereinigten Aldolase mit verschiedenen Reagenzien versetzt und 5 Tage lang bei RT bzw. -20°C gelagert. Tab. 2 zeigt das Ergebnis:

Tabelle 2

|  | RT | -20°C |
|---|---|---|
| Glycerin 10 %ig | 66 % | 90 % |
| F-1,6-BP 5 %ig | 98 % | 100 % |
| Mercapto-ethanol 4 mM | 54 % | 91 % |
| Albumin 5 %ig | 85 % | 99 % |
| Lyophilisat | 100 % | 100 % |

Tab. 2 Aktivität der Aldolase nach 5 Tagen Lagerung

Stabilitätsvergleich mit Kaninchenmuskel-Aldolase

Die Stabilität der erfindungsgemäßen Aldolase wurde mit derjenigen von Kaninchenmuskel-Aldolase verglichen. Dazu wurden beide Enzyme unter den in Tabelle 3 angegebenen aktiven Bedingungen getestet:

Tabelle 3

| | Standard-bedingungen | aktive Bedingungen |
|---|---|---|
| Temperatur | 25°C | 25°C |
| pH | 6,5 | 6,0 |
| Lösungsmittel | bidest. $H_2O$ | DHAP 20 mM |
| | | Glyoxylsäure 75 mM |
| | | in bidest. $H_2O$ |
| Enzymkonz. | 0,27 mg/ml | 0,27 mg/ml |

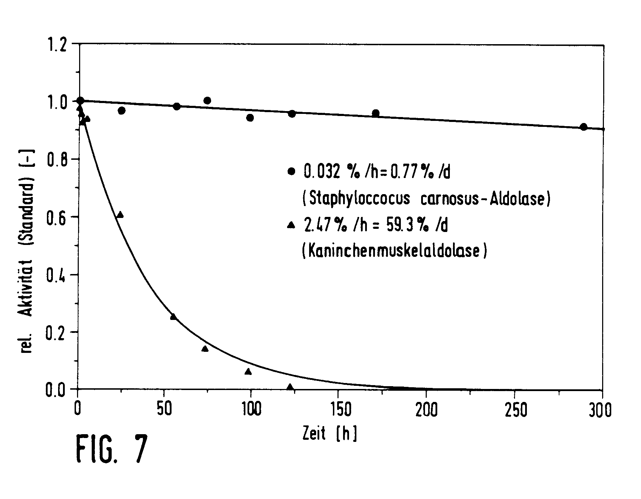

Für beide Versuche wurde eine Enzymkonzentration vergleichbarer Startaktivität eingestellt. Sie betrug für die Kaninchenmuskel-Aldolase 3,4 U/ml (9 U/mg) und für die S. carnosus Aldolase 2,9 U/ml (25 U/mg). Figur 7 zeigt die Stabilität beider Aldolasen über einen Zeitraum von 300 Stunden. Zur Bestimmung der Desaktivierungsrate wurde eine exponentielle Desaktivierung erster Ordnung angenommen, die Desaktivierungsrate ergab sich aus dem exponentiellen Faktor ( 1 = 100 % / Zeiteinheit).

Substratspektrum

Zur Ermittlung des Substratspektrums wurden exemplarisch eine Anzahl von Aldehyden getestet. Dabei wurde folgender Ansatz gewählt:

20 mM DHAP 200 mM Aldehyd 1 - 4 U/ml Aldolase

Nach der Inkubation der Aldolase erfolgte diskontinuierliche DC-Kontrolle. In den Ansätzen mit nachfolgend angegebenen Aldehyden konnte nach 5 - 8 h kein DHAP mehr nachgewiesen werden.

EP 0 431 548 B1

Aldolase-Substrate

| Aldehyd | $R_p$ des Produkts |
|---|---|
| Glycerinaldehyd-3-Phosphat | 0,36 |
| Methylglyoxal | 0,37 |
| D(+)-Glycerinaldehyd | 0,44 |
| L(-)-Glycerinaldehyd | 0,44 |
| D,L-Glycerinaldehyd | 0,44 |
| Glykolaldehyd | 0,66 |
| Phthaldialdehyd | 0,69 |
| Formaldehyd | 0,71 |
| Acetaldehyd | 0,93 |
| Chloracetaldehyd | 1,08 |
| Propionaldehyd | 1,20 |
| 3-Methylmercaptopropionaldehyd | 1,24 |
| Isobutyraldehyd | 1,36 |
| Trimethylacetaldehyd | 1,41 |
| Butyraldehyd | 1,40 |
| 3-Ketobutyraldehyd | 1,48 |
| Isovaleraldehyd | 1,49 |
| Malondialdehyd | 1,49 |
| Pyridin(2)carbaldehyd | 1,50 |
| Pyridin(4)carbaldehyd | 1,50 |
| Phenylacetaldehyd | 1,53 |
| 2-Methylacetaldehyd | 1,54 |
| Valeraldehyd | 1,60 |

Als Beispiel für eine F-1,6-BP-Aldolase katalysierte Reaktion wird nachfolgend die Synthese von 5-6-Dideoxyhexulose beschrieben:

Dihydroxyacetonphosphat (DHAP) wurde im präparativen Maßstab mit Propionaldehyd in Gegenwart von F-1,6-BP-Aldolase bei 25°C umgesetzt.

    Ansatz:    50 mM ( 5 mmol) DHAP
               500 mM (50 mmol) Propionaldehyd
               40 U Aldolase

Der Propionaldehyd wurde vor der Reaktion frisch destilliert und unter Argon aufbewahrt. Der Verlauf der Reaktion wurde sowohl polarimetrisch bei einer Wellenlänge von 595 nm als auch durch die Bestimmung des Rest-DHAP-Gehaltes beobachtet. Nach 6,5 h war die Reaktion beendet, und das Produkt wurde als Cyclohexylammoniumsalz isoliert.

Zur Aufklärung der Struktur wurden 250 mg der Substanz mit saurer Phosphatase dephosphoryliert. Die Peracetylierung des freien Zuckers erfolgte mit Essigsäureanhydrid in Pyridin. Mit Hilfe der $^1$H-bzw. $^{13}$C-NMR Spektren konnte die isolierte Verbindung eindeutig als das erwartete Produkt identifiziert werden.

9

Die erfindungsgemäße Aldolase ist für die Synthese von Kohlenhydraten bzw. Kohlenhydratderivaten außerordentlich nützlich, insbesondere kann sie für die Herstellung von 6-Desoxy-Fructose-1-Phosphat einer Vorstufe des Aromastoffs Furaneol (Wong. u. a. J. Org. Chem. 48 (1983) 3493 - 3497) verwendet werden. Ferner können unter Anwendung der Aldolase höhere Zucker mit ≧ 6 C-Atomen, insbesondere mit $C_8$, $C_9$ usw. erhalten werden (siehe Bednarski u. a. Tetrahedron Lett. (1986) 27 5807 - 5810). Komplizierte Aminozucker wie 1-Desoxymannojirimicin, 1-Desoxynojirimicin und 1,4-Didesoxy-1,4-imino-D-arabinitol, die als wirksame β-Glucosidase-inhibitoren wichtig sind (Ziegler u. a. Angew. Chemie 100 (1988) 737 - 738) werden mit Hilfe der erfindungsgemäßen Aldolase besser zugänglich, die zum Teil mit HIV-Viren hemmender Wirksamkeit begabt sind (siehe Fleet u. a. FEBS Letters 237 (1988) 128-132).

Eine weitere Anwendung für die Herstellung von Pheromonen wird von M. Schultz et al. in Tetrahydron Letters Bd. 31 (1990) Seiten 867-8 angegeben.

## Patentansprüche

1.  Verfahren zur Synthese von Kohlenhydraten bzw. Derivaten derselben durch enzymatische Umsetzung von Aldehyden mit Dihydroxyacetonphosphat in Gegenwart von aus Staphylococcen isolierter Klasse 1-Fructose-1,6-bisphosphat-Aldolase (F-1,6-BP-Aldolase)
    **dadurch gekennzeichnet,**
    daß man eine aus Staphylococcus carnosus isolierte F-1,6-BP-Aldolase verwendet.

2.  Verfahren nach Anspruch 1,
    **dadurch gekennzeichnet,,**
    daß man eine aus Staphylococcus carnosus DSM 20 501 isolierte Aldolase verwendet.

3.  Verfahren zur Gewinnung von F-1,6-BP-Aldolase für eine Synthese nach Anspruch 1 oder 2,
    **dadurch gekennzeichnet,**
    daß man in einem Fermenter angezüchtete Zellmasse von Staphylococcus carnosus von der Kulturflüssigkeit abtrennt und befreit und das Enzym nach Zellaufschluß gewinnt.

4.  Verfahren nach Anspruch 3,
    **dadurch gekennzeichnet,**
    daß man das Enzym nach Zellaufschluß durch fraktionierte Ammoniumsulfatfällung, pH-Fraktionierung und Ionenaustauscherchromatographie isoliert.

5.  Verfahren nach Anspruch 3,
    **dadurch gekennzeichnet,**
    daß man die enzymhaltige Flüssigkeit nach Zellaufschluß einer wäßrigen 2-Phasenextraktion unterwirft und das Enzym aus der Oberphase durch Anionenaustauscher-Chromatographie gewinnt.

6.  Aldolase, erhältlich nach einem der Ansprüche 3 bis 5,
    **gekennzeichnet durch**
    eine 60°C- bzw. 100°C-Stabilität in aufgereinigter Form (≈ 25 U/mg) bei pH 7,5 in 0,06 M Tris-HCl-Pufferlösung in Eppendorfgefäßen entsprechend einer Restaktivität nach 30 Minuten zwischen 70 und 80 % bzw. 20 und 30 %, insbesondere von 76 % bzw. 26 % sowie eine Desaktivierungsrate unter Synthesebedingungen bei T = 25°C; pH 6,5; 20 mM DHAP; 75 mM Glyoxylsäure; Enzymaktivität 2,9 U/ml bzw. 25 U/mg von ≦ 1% d.

## Claims

1.  A method for the synthesis of carbohydrates or derivatives thereof by the enzymatic reaction of aldehydes with dihydroxyacetone phosphate in the presence of class 1 fructose 1,6-bisphosphate aldolase (F-1,6-BP-aldolase) isolated from staphylococci, characterised in that an F-1,6-BP-aldolase isolated from Staphylococcus carnosus is used.

2.  A method according to claim 1, characterised in that aldolase isolated from Staphylococcus carnosus DSM 20 501 is used.

3. A method of obtaining F-1,6-BP-aldolase for a synthesis according to claim 1 or 2, characterised in that a cell mass of Staphylococcus carnosus cultured in a fermenter is separated and freed from the culture liquid and the enzyme is obtained after cell digestion.

4. A method according to claim 3, characterised in that the enzyme is isolated after cell digestion by fractionated ammonium sulphate precipitation, pH fractionation and ion exchange chromatography.

5. A method according to claim 3, characterised in that the enzyme-containing liquid is subjected after cell digestion to an aqueous 2-phase extraction and the enzyme is obtained from the upper phase by anion exchange chromatography.

6. Aldolase, obtainable according to any one of claims 3 to 5, characterised by a stability at 60°C and 100°C in purified form ($\approx$ 25 U/mg) at pH 7.5 in 0.06 M tris-HCl buffer solution in Eppendorf vessels corresponding to a residual activity after 30 minutes between 70 and 80 % and 20 and 30 %, respectively, particularly of 76 % and 26 %, respectively, and by a rate of deactivation under synthesis conditions at T = 25°C; pH 6.5; 20 mM DHAP; 75 mM glyoxylic acid; enzyme activity 2.9 U/ml and 25 U/mg, respectively, of $\leq$ 1 % d.

**Revendications**

1. Procédé de synthèse des carbohydrates et de leurs dérivés par réaction enzymatique d'aldéhydes avec le phosphate de dihydroxyacétone en présence de fructose-1,6-bisphosphate-aldolase (F-1,6-BP-aldolase) de la classe 1, isolée à partir de staphylocoques, caractérisé en ce qu'on utilise une F-1,6-BP-aldolase isolée du *Staphylococcus carnosus*.

2. Procédé selon la revendication 1, caractérisé en ce qu'on utilise une aldolase isolée de la souche DSM 20 501 de *Staphylococcus carnosus*.

3. Procédé de préparation de F-1,6-BP-aldolase pour une synthèse selon la revendication 1 ou 2, caractérisé en ce qu'on sépare et on libère du bouillon de culture une masse de cellules de *Staphylococcus carnosus* mise en culture dans un fermentateur et on obtient l'enzyme après la décomposition des cellules.

4. Procédé selon la revendication 3, caractérisé en ce qu'on isole l enzyme, après la décomposition des cellules, par précipitation fractionnée avec du sulfate d'ammonium, par fractionnement fondé sur le pH et par chromatographie échangeuse d'ions.

5. Procédé selon la revendication 3, caractérisé en ce qu'on soumet le liquide contenant l'enzyme, après la décomposition des cellules, a une extraction à 2 phases en milieu aqueux et on recueille l'enzyme dans la phase supérieure par chromatographie échangeuse d'anions.

6. Aldolase pouvant être obtenue selon l'une des revendications 3 à 5, caractérisée par une stabilité à 60°C et 100°C sous une forme purifiée ($\approx$25 U/mg), à pH 7,5, dans une solution tamponnée de Tris-HCl 0,06M, dans des récipients d'Eppendorf, correspondant à une activité résiduaire comprise respectivement entre 70 et 80 % et 20 et 30 %, en particulier de 76 % et 26 %, après 30 minutes, ainsi qu'un taux de désactivation $\leq$ 1 %/d dans les conditions de synthèse de T = 25°C ; pH 6,5 ; 20 mM DHAP ; 75 mM d'acide glyoxylique ; activité enzymatique de 2,9 U/ml ou 25 u/mg.

FIG. 1

FIG. 2

FIG. 3

FIG. 4

FIG. 5

FIG. 6

FIG. 7